# EUROPEAN PATENT SPECIFICATION

(11) **EP 2 718 862 B1**
(45) Date of publication and mention of the grant of the patent: **31.10.2018**
(21) Application number: 12730271.9
(22) Date of filing: 24.05.2012
(51) Int. Cl.: G06F 19/22

(54) **METHOD FOR ASSEMBLY OF NUCLEIC ACID SEQUENCE DATA**
VERFAHREN ZUM ZUSAMMENSETZEN VON NUKLEINSÄURESEQUENZDATEN
PROCÉDÉ POUR L'ASSEMBLAGE DES DONNÉES DE SÉQUENCE D'ACIDE NUCLÉIQUE

(30) Priority: 06.06.2011 US 201161493541 P
(43) Date of publication of application: 16.04.2014
(73) Proprietor: Koninklijke Philips N.V., 5656 AE Eindhoven (NL)
(72) Inventor: KUMAR, Sunil, NL-5656 AE Eindhoven (NL); SINGH, Randeep, NL-5656 AE Eindhoven (NL); DIMITROVA, Nevenka, NL-5656 AE Eindhoven (NL)
(74) Representative: Steffen, Thomas
(86) International application number: PCT/IB2012/052613
(87) International publication number: WO 2012/168815

(56) References cited:
- M. POP: "Genome assembly reborn: recent computational challenges", BRIEFINGS IN BIOINFORMATICS, vol. 10, no. 4, 1 July 2009 (2009-07-01), pages 354-366, XP0055047552, ISSN: 1467-5463, DOI: 10.1093/bib/bbp026
- MILLER JASON R ET AL: "Assembly algorithms for next-generation sequencing data", GENOMICS, ACADEMIC PRESS, SAN DIEGO, US, vol. 95, no. 6, 1 June 2010 (2010-06-01), pages 315-327, XP009165741, ISSN: 0888-7543
- D. S. HORNER ET AL: "Bioinformatics approaches for genomics and post genomics applications of next-generation sequencing", BRIEFINGS IN BIOINFORMATICS, vol. 11, no. 2, 1 March 2010 (2010-03-01), pages 181-197, XP055047553, ISSN: 1467-5463, DOI: 10.1093/bib/bbp046
- SHENDURE J ET AL: "Next-generation DNA sequencing", NATURE BIOTECHNOLOGY, NATURE PUBLISHING GROUP, NEW YORK, NY, US, vol. 26, no. 10, 1 October 2008 (2008-10-01), pages 1135-1145, XP002572506, ISSN: 1087-0156, DOI: 10.1038/NBT1486 [retrieved on 2008-10-09]
- PAUL FLICEK ET AL: "Sense from sequence reads: methods for alignment and assembly", NATURE METHODS, NATURE PUBLISHING GROUP, GB, vol. 6, no. 11s, 1 December 2009 (2009-12-01), pages s6-s12, XP008148167, ISSN: 1548-7091, DOI: 10.1038/NMETH.1376 [retrieved on 2009-10-15]

## Description

### FIELD OF THE INVENTION

The present invention relates to a method for assembly of nucleic acid sequence data comprising nucleic acid fragment reads into (a) contiguous nucleotide sequence segment(s), comprising the steps of: (a) obtaining a plurality of nucleic acid sequence data from a plurality of nucleic acid fragment reads; (b) aligning said plurality of nucleic acid sequence data to a reference sequence;(c) detecting one or more gaps or regions of non-matching with the reference sequence in the alignment output of step (b), wherein prior to the aligning step (b) a masking out of nucleic acid sequence data relating to known polymorphisms, highly variable regions, disease related mutations or modifications, repeats, low mapability regions, CPG islands, or regions with specific biophysical features is performed;(d) performing *de novo* sequence assembly of nucleic acid sequence data mapping to said gaps or regions of non-matching; and (e) combining the alignment output of step (b) and the assembly output of step (d) in order to obtain (a) contiguous nucleotide sequence segment(s). The present invention further relates to a method wherein the detection of gaps or regions of non-matching is performed by implementing a base quality, coverage, complexity of the surrounding region, or length of mismatch filter or threshold. Also envisaged is the masking out of nucleic acid sequence data relating to known polymorphisms, disease related mutations or modifications, repeats, low mapability regions, CPG islands, or regions with certain biophysical features. In addition, a corresponding program element or computer program for assembly of nucleic acid sequence data and a sequence assembly system for transforming nucleic acid sequence data comprising nucleic acid fragment reads into (a) contiguous nucleotide sequence segment(s) is provided.

### BACKGROUND OF THE INVENTION

With the introduction of next generation or ultra-high-throughput sequencing techniques the amount of sequence data has increased enormously, while the costs for obtaining sequence information and the time needed for the provision of this information have been dramatically reduced and will be further decreased in the future. Research, as well as clinical applications of next generation sequencing approaches will have an impact on transcriptome analysis and gene annotation, allow RNA splice identification, SNP discovery or genome methylation analysis and provide a way to identify the etiology of diseases, and to screen for genomic pattern on a personal basis.

The next generation sequencing (NGS) is currently based on only a handful of platforms including the Roche/454, the Illumina/Solex and the ABI SOLiD systems. The underlying technology relies on a template amplification step before the sequencing starts. In consequence, the read length is shortened in comparison to the traditional Sanger-based technology: whereas the de-deoxy terminator approach provided read lengths of 650 to 800 bp, NGS approaches have read lengths of 35 - 400 bp (Bao et al., Journal of Human Genetics, 28 April 2011, p. 1-9). Furthermore, the raw data obtained from the NGS platforms is not standardized and shows differences in read lengths, error profiles, matching thresholds etc. Thus, the implementation of NGS approaches connotes an increase in amount and complexity of sequence information.

However, the output of NGS sequence machines is essentially worthless by itself, since the sequence reads only become meaningful upon a reconstruction of the underlying contiguous genomic sequence. Furthermore, for routine uses of NGS, e.g. in clinical setups, a high sequence accuracy and an expedient way to select genomic subsets of interest are of importance. Upon a higher integration of genome sequencing into the practice of medical counseling, there will be an increased responsibility of geneticists to ensure that the information obtained is in fact true and represents the original genome of the individual.

There is, thus, a need for a method allowing the accurate and timesaving alignment and assembly of nucleic acid sequence data as derivable from NGS approaches.

### SUMMARY OF THE INVENTION

The present invention addresses this need and provides means and methods, which allow the assembly of nucleic acid sequence data comprising nucleic acid fragment reads into contiguous nucleotide sequence segments. The above objective is in particular accomplished by a computer-implemented method comprising the steps of:
(a) obtaining a plurality of nucleic acid sequence data from a plurality of nucleic acid fragment reads;
(b) aligning said plurality of nucleic acid sequence data to a reference sequence;
(c) detecting one or more gaps or regions of non-matching with the reference sequence in the alignment output of step (b); wherein prior to the aligning step (b) a

masking out of nucleic acid sequence data relating to known polymorphisms, highly variable regions, disease related mutations or modifications, repeats, low mapability regions, CPG islands, or regions with specific biophysical features is performed;
(d) performing *de novo* sequence assembly of nucleic acid sequence data mapping to said gaps or regions of non-matching; and
(e) combining the alignment output of step (b) and the assembly output of step (d) in order to obtain (a) contiguous nucleotide sequence segment(s).

This method provides the advantage that a bias, which is typically generated when a reference sequence alignment is performed, can be overcome by using *de novo* assembly steps. Furthermore, typical problems associated with the filling of the gaps that are created during reference sequence alignment, polymorphism lengths detection and in particular the fitting of un-aligned sequence in the consensus assembly may be solved when closing these information gaps or breaks via *de novo* assembly. At the same time, annotation problems known from *de novo* assembly approaches can be mitigated by basing parts of the analysis on a reference sequence. The method accordingly starts with a reference sequence alignment and when it finds a gap or regions of non-matching it switches to *de novo* alignment, e.g. until it again detects the reference alignment. This creates a consensus assembly or contiguous nucleotide sequence segments with a significantly increased sequence accuracy. In fact, the accordingly assembled sequence represents individual genomes rather than reference genomes and avoids reference sequence associated bias problems. The presently described method is accordingly assumed to have huge implications, inter alia in medical genetics where it may help in determining the genetic basis of complex genetic disorders.

In a preferred embodiment of the present invention, wherein the above mentioned plurality of nucleic acid sequence data is converted to a unified format.

In another preferred embodiment of the present invention the detection of step (c) as mentioned herein above is performed by implementing a filter or threshold.

In further preferred embodiments, said filter or threshold is a base quality, coverage, complexity of the surrounding region or length of mismatch filter or threshold.

In a particularly preferred embodiment said masked out nucleic acid sequence data is subjected to a *de novo* sequence assembly of step (d) as mentioned herein above.

In another preferred embodiment of the present invention the above defined step (b) is carried out with a reference alignment algorithm. In a particularly preferred embodiment of said reference alignment algorithm is BFAST, ELAND, GenomeMapper, GMAP, MAQ, MOSAIK, PASS, SeqMap, SHRiMP, SOAP, SSAHA, or CLD. Even more preferred is Bowtie or BWA.

In yet another preferred embodiment of the present invention, the above defined step (c) is carried out with a *de novo* assembly algorithm. In a particularly preferred embodiment of said *de novo* assembly algorithm is AAPATHS, Edena, EULER-SR, MIRA2, SEQAN, SHARCGS, SSAKE, SOAPdenovo, VCAKE. Even more preferred is ABySS or Velvet.

In a further preferred embodiment the herein above mentioned reference sequence is an essentially complete prokaryotic, eukaryotic or viral genome sequence, or a sub-portion thereof. In a particularly preferred embodiment of the present invention said reference sequence is a human genome sequence, an animal genome sequence, a plant genome sequence, a bacterial genome sequence, or a sub-portion thereof.

In a further preferred embodiment of the present invention said reference sequence is selected from a group or taxon, which is phylogenetically related to the organism, whose nucleic acid sequence data is to be assembled.

In yet another preferred embodiment of the present invention said reference sequence is a genomic sub-portion having regulatory potential selected from the group comprising exon sequences, promoter sequences, enhancer sequences, transcription factor binding sites, or any grouping or sub-grouping thereof.

In a further preferred embodiment said reference sequence is a virtual sequence based on sequence composition parameters, or based on biophysical nucleic acid properties. In a particularly preferred embodiment of the present invention said composition parameter is the presence of monomers, dimers and/or trimers. In a further preferred embodiment of the present invention said biophysical nucleic acid property is the stacking energy, the presence of propeller twist, the bendability of the nucleic acid, duplex stability, the amount of disrupt energy, the amount of free energy, the presence of DNA denaturation or DNA bending stiffness.

In a further aspect the present invention relates to a program element or computer program for assembly of nucleic acid sequence data comprising nucleic acid fragment reads into contiguous nucleotide sequence segments, which when being executed by a processor is adapted to carry out the steps of a method as defined herein above.

In yet another aspect the present invention relates to a sequence assembly system for transforming nucleic acid sequence data comprising nucleic acid fragment reads into (a) contiguous nucleotide sequence segment(s), comprising a computer processor, memory, and (a) data storage device(s), the memory having programming instructions to execute a program element or computer program as defined herein above.

In a preferred embodiment of the present invention said sequence assembly system is associated or connected to a sequencer device. In a further preferred embodiment said sequence assembly system is a medical decision support system. In a particularly preferred embodiment said medical decision support system is a diagnostic decision support system.

### BRIEF DESCRIPTION OF THE DRAWINGS

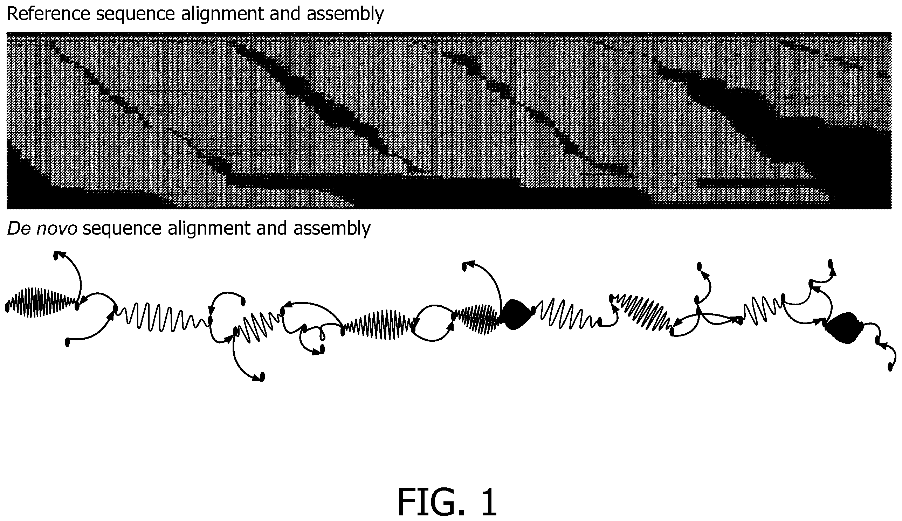

Fig. 1 provides an overview over reference and *de novo* sequence and alignment procedures. Reference sequence alignment and assembly shows mapping of reads to the reference sequences. *De novo* assembly shows the generation of contigs using ABySS algorithm based on an excerpt from an ABySS-Explorer view, where edges represent contigs and nodes represent common *k*-1-mers between adjacent contigs. The labels correspond to SET contig IDs. Contig lengths and coverage are indicated by the length and the thickness of the edges, respectively. Arrows and edge arc shape indicate the direction of contigs and the polarity of the nodes distinguish reverse complements of common *k*-1-mers between adjacent contigs.

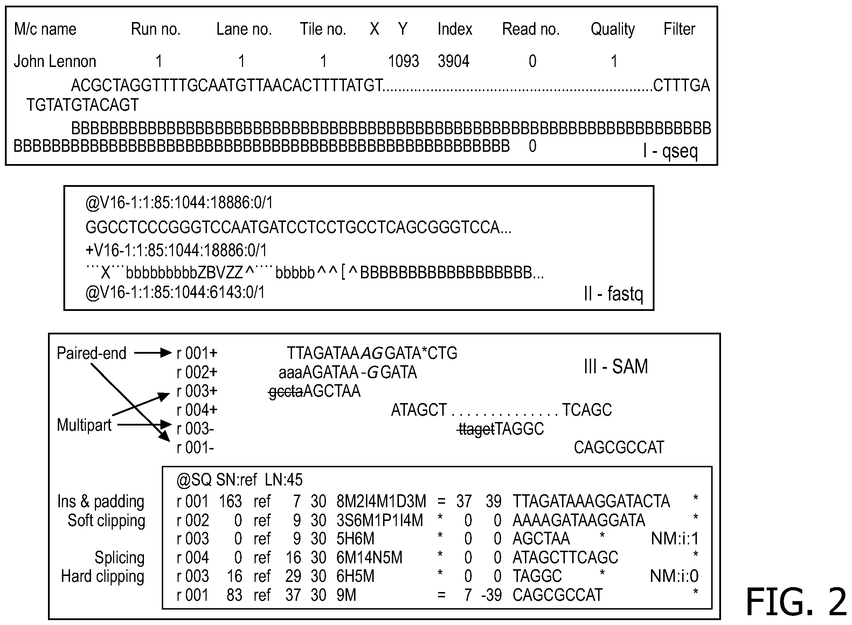

Fig. 2 shows examples of different sequence file formats. Depicted are the qseq format (sequence read output from Illumina instrument which has machine, run and quality information), the fastq format (Illumina read name, sequence and quality which has been derived from qseq file) and SAM format (Sequence Alignment/ Map) which is output of BWA aligner. The SAM format, which allows to store read alignment information against a reference.

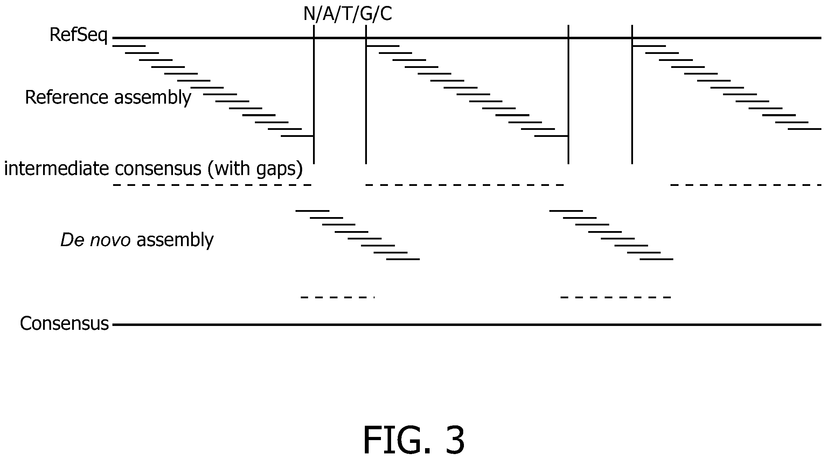

Fig. 3 depicts an overview over the alignment and assembly steps according to the present invention. It shows the overall method of combining reference alignment and *de novo* assembly. Initially the reads are aligned to a reference sequence. Where ever a gap (e.g. user defined size, ex: >10base) of N/A/T/G/C is identified where the reads are not matching to the reference in continuation with the previous read in an overlap fashion, the *de novo* assembly will be started. There will be a *de novo* contig formation until the next read matching to the reference is identified. This *de novo* contig will then be merged with intermediate consensus to give final consensus sequence.

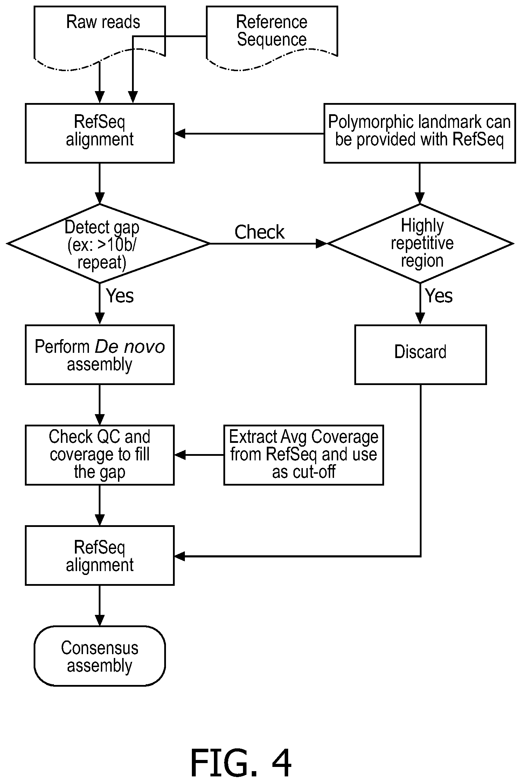

Fig. 4 shows a process chart of method steps of a combination of reference sequence alignment and *de novo* assembly according to the present invention.

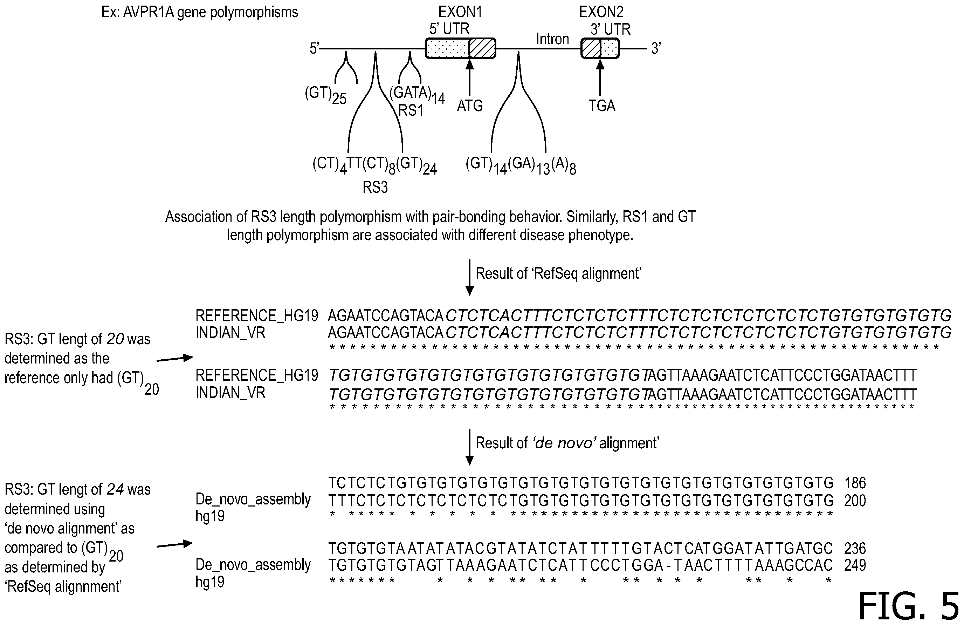

Fig. 5 depicts the determination of the exact length of GT polymorphism in AVPR1A gene using a combination of reference alignment and *de novo* assembly following the method according to the present invention. First, reads with the reference genome to extract the AVPR1 gene for the analyzed sample were aligned. As the RS3 is highly polymorphic site and is associated with clinical phenotype, a *de novo* assembly of the reads that were falling in this chromosome was carried out and subsequently contigs were generated. After obtaining the contigs relaxed sequence alignment (allowing mismatch and gaps) was performed to merge the *de novo* contig with the reference consensus. The obtained consensus sequence showed the true polymorphic repeat for the analyzed sample.

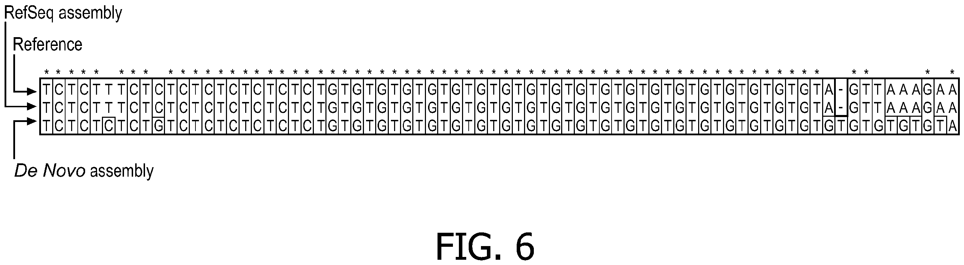

Fig. 6 shows a direct comparison between the Reference Sequence assembly and the *de novo* assembly of the AVPR1A gene. Reads were aligned to reference and *de novo* assembly was performed. The consensus generated from reference was then aligned against *de novo* contig using ClustanW. Shown is a difference in GT repeats which is biased from reference as compared to *de novo* displaying different repeat contents.

### DETAILED DESCRIPTION OF EMBODIMENTS

The inventors have developed means and methods, which allow the assembly of nucleic acid sequence data comprising nucleic acid fragment reads into contiguous nucleotide sequence segments.

Although the present invention will be described with respect to particular embodiments, this description is not to be construed in a limiting sense.

Before describing in detail exemplary embodiments of the present invention, definitions important for understanding the present invention are given.

As used in this specification and in the appended claims, the singular forms of "a" and "an" also include the respective plurals unless the context clearly dictates otherwise.

In the context of the present invention, the terms "about" and "approximately" denote an interval of accuracy that a person skilled in the art will understand to still ensure the technical effect of the feature in question. The term typically indicates a deviation from the indicated numerical value of ±20 %, preferably ±15 %, more preferably ±10 %, and even more preferably ±5 %.

It is to be understood that the term "comprising" is not limiting. For the purposes of the present invention the term "consisting of' is considered to be a preferred embodiment of the term "comprising of'. If hereinafter a group is defined to comprise at least a certain number of embodiments, this is meant to also encompass a group which preferably consists of these embodiments only.

Furthermore, the terms "first", "second", "third" or "(a)", "(b)", "(c)", "(d)" etc. and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

In case the terms "first", "second", "third" or "(a)", "(b)", "(c)", "(d)" etc. relate to steps of a method or use there is no time or time interval coherence between the steps, i.e. the steps may be carried out simultaneously or there may be time intervals of seconds, minutes, hours, days, weeks, months or even years between such steps, unless otherwise indicated in the application as set forth herein above or below.

It is to be understood that this invention is not limited to the particular methodology, protocols, reagents etc. described herein as these may vary. It is also to be understood that the terminology used herein is for the purpose of describing particular embodiments only, and is not intended to limit the scope of the present invention that will be limited only by the appended claims. Unless defined otherwise, all technical and scientific terms used herein have the same meanings as commonly understood by one of ordinary skill in the art.

As has been set out above, the present invention concerns in one aspect a method for assembly of nucleic acid sequence data comprising nucleic acid fragment reads into (a) contiguous nucleotide sequence segment(s), comprising the steps of:
(a) obtaining a plurality of nucleic acid sequence data from a plurality of nucleic acid fragment reads;
(b) aligning said plurality of nucleic acid sequence data to a reference sequence;
(c) detecting one or more gaps or regions of non-matching with the reference sequence in the alignment output of step (b);
(d) performing *de novo* sequence assembly of nucleic acid sequence data mapping to said gaps or regions of non-matching; and
(e) combining the alignment output of step (b) and the assembly output of step (d) in order to obtain (a) contiguous nucleotide sequence segment(s).

The term "assembly" of nucleic acid sequence data as used herein refers to the arrangement of singularly or independently provided sequence data into a contiguous nucleotide sequence segment. The term "contiguous nucleotide sequence segment(s)" as used herein refers to the output of the presently claimed method being a coherent, non-redundant and preferably error-free or substantially error-free sequence context. A "sequence segment" as used herein may be any stretch comprising more the information content of more than about 50 reads. Preferably, a sequence segment may be an entire genome, an entire chromosome, a chromosome arm, one or more sub-portion of a chromosome, a conjunction of interrelated sequence, e.g. exomes, transcriptome-related sequences, a conjunction of open reading frames, introns, transposon-sequences, repeats, regulome-related sequences such as transcription factor binding sites, methylation binding protein sites, specific regions with higher probability of Histone 3 lysine 4 mono- di- and tri-methylation etc. A "nucleic acid fragment read" as used herein refers to a single, short contiguous information piece or stretch of sequence data. A read may have any suitable length, preferably a length of between about 30 nucleotides to about 1000 nucleotides. The length generally depends on the sequencing technology used for obtaining it. In specific embodiments, the reads may also be longer, e.g. 2 to 10 kb or more. The present invention generally envisages any read or read length and is not to be understood as being limited to the presently available read lengths, but also includes further developments in this area, e.g. the development of long reading sequencing approaches etc.

In a first step of the method, a plurality of nucleic acid sequence data from a plurality of nucleic acid fragment reads may be obtained. A "nucleic acids sequence data" as used herein may be any sequence information on nucleic acid molecules known to the skilled person. The sequence data preferably includes information on DNA or RNA sequences, modified nucleic acids, single strand or duplex sequences, or alternatively amino acid sequences, which have to converted into nucleic acid sequences. The sequence data may additionally comprise information on the sequencing machine, date of acquisition, read length, direction of sequencing, origin of the sequenced entity, neighbouring sequences or reads, presence of repeats or any other suitable parameter known to the person skilled in the art. The sequence data may be presented in any suitable format, archive, coding or document known to the person skilled in the art. The data may, for example, be in the format of FASTQ, Qseq, CSFASTA, BED, WIG, EMBL, Phred, GFF, SAM, SRF, SFF or ABI-ABIF, as depicted and further explained in the following Table 1:

**Table 1:**

| **File Format** | **Developed By** | **Used By** | **Extension** | **Representation** | **Remarks** |
|---|---|---|---|---|---|
| FAS TQ | Sanger Institute | Illumina, Sanger | .fq,.fastq, .txt | Text Based for sequence and quality score | -Simple (Fasta like) *-de facto* standard for many sequencing instruments like fastq-Sanger, fastq-Solexa,fastq-illumina -Encode Phred quality score using ASCII |
| Qseq | Illumina | Illumina | .qseq | Sequence and Quality score | -A single file will be created for each lane |
| CSF AST A | ABI | ABI | .csfasta | Color-Space sequence reads | -Conversion from color-space to base-space leads to error propagation -Better visualization and alignment. |
| BED | UCSC Genome Bioinformatics Group | Genome Browsers | .bed,.txt | Text based | - Flexible way to define the data lines that are displayed in an annotation track -Accepted by most genomic browsers |
| WIG | UCSC Genome Bioinformatics Group | Genome Browsers | .wig,.txt | Genome browser track format | - Display of continuous-valued data in a track format |
| EMBL | European Molecular Biology Laboratory | EMBL, GenBank databases | .embl,.em | Several sequences in a single file | - Represents database records for nucleotide and peptide sequences from EMBL databases -Meta information can be optimally stored. |
| Phred | Research Output | All sequencing projects | .phd | Store serialized chromatogram data | -Widely used in storing quality scores for bases |
| GFF | Sanger Institute | GMOD | .gff | Genomic feature in a text file | - Data exchange and representation of genomic data |
| SAM | Collaborative result of several major genome centres | All sequencing projects | .sam | generic nucleotide alignment format | -Support longer reads and alignment with more than one indels. -Used by the 1000Genome Project Committee -Simple, Compact in size |
| SRF | Developed using an open process various | All sequencing technologies | .srf | Generic binary format for DNA sequence data | -Format flexible to store data from different DNA sequencing technologies. |
| SFF | 454(Roche) | 454 FLX | .sff | Binary container file to encode results from 454 FLX | -Can store one or more than one reads from 454 life sciences platform. |
| ABI-ABIF | ABI | SOLiD(ABI) | .abl,.fsa | Binary chromatogram Files | -Accommodate heterogeneous data - Stores data directory wise. |

Preferably, the data or data sets are present in one data format, more preferably in a unified data format, e.g. in the fastq format, along with their base quality either in Phred / Phrap or modified format. It is further preferred that the data format at least covers the sequence read and its associated base quality.

In a particularly preferred embodiment of the present invention, the plurality of sequence data may be converted into a unified format. Such a conversion may be carried out by any suitable conversion tool known to the person skilled in the art, for example standard conversion tools which are capable of converting an Illumina format into a Sanger format, which may be used by several alignment algorithms, or any other comparable tool capable of converting a format indicated in Table 1 into another format indicated in Table 1 or known to the person skilled in the art. The conversion may be performed such that at least a minimum amount of essential data is kept. Such a minimum amount of data may comprise, for example, the sequence itself, the run information, paired end library information, mate pair library information, single end library information, and base QC value. The preferred format into which the sequence data may be converted is any suitable format, which is recognized by reference sequence alignment algorithms, as well as *de novo* assembly algorithms. A preferred example is the fastq format. Alternatively, the sequence data may also be converted into the cfasta/SCARF format. The present invention further envisages any further, e.g. newly defined or developed format being able to be used by both, reference sequence alignments and *de novo* assembly procedures.

The data may comprise single entries or multiple entries within one data set. The data may also include one or more data sets, or a plurality of data sets. The term "plurality" as used herein accordingly refers to one or more data sets coming from one or more origins or sources. The data sets or data may, for example, have the same format and/or come from the same origin, e.g. the same sequencing machine, the same patient or subject or have been obtained with the same sequencing technology, or they may have different formats and/or come from different origins such as different sequencing machines or different patients or subjects or have been obtained with different sequencing technologies.

The term "obtaining sequence data from a plurality of nucleic acid fragment reads" as used herein refer to the process of determining the sequence information of a subject, or a group of subjects by the performance of nucleic acid sequencing reactions, The present invention in one alternative embodiment uses previously obtained sequence data, e.g. derivable from databases, external sequencing projects, laboratories, archives etc. In another alternative embodiment the present invention also envisages the step of obtaining the sequence data as an integral part of method step (a).

Methods for sequence determination are generally known to the person skilled in the art. Preferred are next generation sequencing methods or high throughput sequencing methods. For example, a subject's, group of subject's, or population's genomic sequence may be obtained by using Massively Parallel Signature Sequencing (MPSS). An example of an envisaged sequence method is pyrosequencing, in particular 454 pyrosequencing, e.g. based on the Roche 454 Genome Sequencer. This method amplifies DNA inside water droplets in an oil solution with each droplet containing a single DNA template attached to a single primer-coated bead that then forms a clonal colony. Pyrosequencing uses luciferase to generate light for detection of the individual nucleotides added to the nascent DNA, and the combined data are used to generate sequence read-outs. Yet another envisaged example is Illumina or Solexa sequencing, e.g. by using the Illumina Genome Analyzer technology, which is based on reversible dye-terminators. DNA molecules are typically attached to primers on a slide and amplified so that local clonal colonies are formed. Subsequently one type of nucleotide at a time may be added, and non-incorporated nucleotides are washed away. Subsequently, images of the fluorescently labeled nucleotides may be taken and the dye is chemically removed from the DNA, allowing a next cycle. Yet another example is the use of Applied Biosystems' SOLiD technology, which employs sequencing by ligation. This method is based on the use of a pool of all possible oligonucleotides of a fixed length, which are labeled according to the sequenced position. Such oligonucleotides are annealed and ligated. Subsequently, the preferential ligation by DNA ligase for matching sequences typically results in a signal informative of the nucleotide at that position. Since the DNA is typically amplified by emulsion PCR, the resulting bead, each containing only copies of the same DNA molecule, can be deposited on a glass slide resulting in sequences of quantities and lengths comparable to Illumina sequencing. A further method is based on Helicos' Heliscope technology, wherein fragments are captured by polyT oligomers tethered to an array. At each sequencing cycle, polymerase and single fluorescently labeled nucleotides are added and the array is imaged. The fluorescent tag is subsequently removed and the cylce is repeated. Further examples of sequencing techniques encompassed within the methods of the present invention are sequencing by hybridization, sequencing by use of nanopores, microscopy-based sequencing techniques, microfluidic Sanger sequencing, or microchip-based sequencing methods. The present invention also envisages further developments of these techniques, e.g. further improvements of the accuracy of the sequence determination, or the time needed for the determination of the genomic sequence of an organism etc.

A genomic sequence or a sub-genomic sequence or any portion thereof, e.g. a single nucleic acid fragment read, may be obtained in any suitable quality or accuracy. Preferably, an obtained genomic sequence or a sub-genomic sequence or portion thereof may have no more than one error in every 10,000 bases, in every 50,000 bases, in every 75,000 based, in every 100,000 bases. More preferably, an obtained genomic sequence or a sub-genomic sequence or portion thereof may have no more than one error in every 150,000 bases, 200,000 bases or 250.000 bases. In a further specific embodiment, the obtained genomic sequence or sub-genomic sequence or portion thereof may have an average read depth per haploid genome of at least about 2x, 3x, 4x, 5x, 7x, 10x, 15 x, 20 x, 25 x, 30 x, 35 x, 40 x or more, or any other average depth between 2 x and 50 x, or more. The present invention also envisages the preparation or use of sequences having a higher coverage due to improvements in the sequencing technology. The present invention is accordingly not bound by any error margins or coverage limits, and instead focuses on the implementation of the sequence information available, prepared and obtained according to suitable contemporary sequencing techniques. In a second step of the method, the plurality of nucleic acid sequence data is aligned to a reference sequence. The term "aligning to a reference sequence" as used herein refers to the comparison of nucleic acid fragment read information and their arrangement with an already existing genomic or sub-genomic sequence, preferably followed by a placement of said sequence read stretches within a scaffold provided by the preexisting genomic or sub-genomic sequence. The "reference sequence" as used herein may be any suitable preexisting sequence covering the stretch, which is identical or similar to the newly obtained sequence data or nucleic acid fragment reads.

In preferred embodiments of the present invention, the reference sequence is an essentially complete prokaryotic genome sequence. In further preferred embodiments of the present invention the reference sequence is an essentially complete eukaryotic genome sequence. In yet another preferred embodiment of the present invention said reference sequence is an essentially complete viral genome sequence. Examples of prokaryotic genome sequences are bacterial genome sequences as provided or derivable from NCBI's microbial genome project database. Further details may be derived from McNeil LK et al., The National Microbial Pathogen Database Resource (NMPDR): a genomics platform based on subsystem annotation, Nucleic Acids Res., 2007; 35 (Database issue): D347-53. Examples of eukaryotic genome sequences are provided or derivable from NCBI's BioProject or GenomeProject database, e.g. databases provided under http://www.ncbi.nlm.nih.gov/ bioproject, which also may include data from the 1000 Genomes project (http://www.ncbi.nlm.nih.gov/bioproject/61209), or the ENCODE project (http://www.ncbi.nlm.nih.gov/bioproject/30707. Examples of viral genome sequences are provided or derivable from NCBI's viral genome resources database, or from Belshaw R et al., The RNA Virus Database, Nucleic Acids Res., 2009; 37 (Database issue): D431-D43 5. Particularly preferred is an animal genome sequence, e.g. the genome sequence of domestic or farm animals, e.g. of cat, dog, sheep, cattle, swine, chicken, monkey, rat or mouse. In further preferred embodiments, the genome sequence is a plant genome sequence, e.g. an agricultural crop or fruit, such as corn, potato, wheat, sorghum, rice, cotton, barley, canola, cucumber, soybean, peach, tomato, papaya, or a research model plant such as Arabidopsis thaliana or Brachypodium distachyon etc. Further details and reference sequence information may be derived from any suitable database, e.g. the PlantGDB database. Particularly preferred is a human genome sequence. Examples are population specific genome sequences, e.g. Caucasian genome sequences, African genome sequences, Asian genome sequences etc. Further examples include subject-specific genome sequences, or consensus sequences thereof, e.g. a master reference sequence comprising a conjunction of individual genome sequences. Further details and reference sequence information may be derived from any suitable database, e.g. the UCSC genome database, or the NCBI human genome resources database. These genome sequences may be essentially complete or comprise sub-portions of an essentially complete prokaryotic, eukaryotic, or viral genome as defined above.

The term "essentially complete" as used herein refers to the presence of sequence information on all portions of the genome present in nature. For example, the genome sequence may comprise redundant sequences, repeats, telomeric sequences etc. For example, about 99%, 98%, 97%, 95%, 90%, 85%, 80%, or 75% of the genome sequence may be comprised in an essentially complete genome. In further embodiments, the reference sequence may not comprise certain sequence elements, such as repeats, telomeric sequences, transposon sequences, redundant sequences etc.

A "sub-portion" of an essentially complete genome may, for example, be any percentage of the entire genomic sequence, e.g. 10%, 20%, 30%, 40%, 60%, 60%, 70%, 75% etc. or any value in between. A sub-portion may also be a single chromosome sequence, a chromosomal arm, a combination of more than one chromosome, a haploid chromosomal set etc.

In a further preferred embodiment of the present invention the reference sequence as mentioned herein above may be selected from a group or taxon, which is phylogenetically related to the organism, whose nucleic acid data is to be assembled. Generally, a phylogenetically related organism may have an overall genomic identity of at least about 50%, 60%, 70%, 80%, 90% or 95%. A phylogenetically related organism may, for example, be a monkey in reference to human sequence reads to be analyzed, or vice versa; or barley in reference to wheat or corn reads to be analyzed, or vice versa; or C. glutamticum in reference to E. coli sequence reads to be analyzed, or vice versa etc. In further embodiments, a reference sequence derived from a phylogenetically related organism may comprise a sub-portion of the entire genomic sequence, e.g. comprise certain chromosomes, chromosome combinations, chromosome arms, sections of the genome etc. as defined herein above.

In yet another preferred embodiment of the present invention said reference sequence may comprise a genomic sub-portion having regulatory potential. The term "regulatory potential" as used herein refers to any section of the genome which is involved in transcriptional, epigenetic, structural, mitotic, meiotic, recombinatorial or any other know regulation known to the person skilled in the art. Examples of genomic sub-portions having regulatory potential are exon sequences, promoter sequences, enhancer sequences and transcription factor binding sites. Further envisaged are any groupings or sub-groupings thereof.

Accordingly, a reference sequence may preferably comprise data on all exons present in a genome, preferably a genome as mentioned herein above, more preferably the human genome. The reference sequence may, in further embodiments, comprise information on certain exons, e.g. exons of genetic or biochemical pathway members, exons present on specific chromosomes, chromosome arms, or in specific regions of the genome.

Alternatively, or in addition, a reference sequence may comprise data on all promoter sequences present in a genome, preferably a genome as mentioned herein above, more preferably the human genome. The reference sequence may, in further embodiments, comprise information on certain promoter sequences, e.g. specific promoter sequences of genetic or biochemical pathway members, promoter sequences of genes present on specific chromosomes, chromosome arms, or in specific regions of the genome etc.

Alternatively, or in addition, a reference sequence may comprise data on all enhancer sequences present in a genome, preferably a genome as mentioned herein above, more preferably the human genome. The reference sequence may, in further embodiments, comprise information on certain enhancer sequences, e.g. specific enhancer sequences of genetic or biochemical pathway members, enhancer sequences of genes present on specific chromosomes, chromosome arms, or in specific regions of the genome etc.

Alternatively, or in addition, a reference sequence may comprise data on all transcription factor binding sites present in a genome, preferably a genome as mentioned herein above, more preferably the human genome. The reference sequence may, in further embodiments, comprise information on microRNAs, or noncoding RNAs, or on certain transcription factor binding sites, e.g. specific transcription factor binding sites of genetic or biochemical pathway members, transcription factor binding sites of genes present on specific chromosomes, chromosome arms, or in specific regions of the genome etc.

In yet another embodiment of the present invention, said reference sequence is a virtual sequence. The term "virtual sequence" as used herein refers to a sequence, which is not primarily based on nucleotide identity parameters, but mostly includes one or more different parameters associated with a genome or a sub-portion thereof. These parameters may be associated with each other, or be associated with other non-nucleotide identity parameters, or, in specific embodiments, be associated with nucleotide identity parameters. Examples of parameters to be used for the construction of a virtual sequence are sequence composition parameters. Such "sequence composition parameters" include factors contributing to the composition of the genomic sequence without constituting genetic information per se. Preferred examples are nucleotide monomers, e.g. stretches of two, three, four, 5, 6, 7, 8, 9, 10 or more of A, C, T, or G; or nucleotide dimers or stretches of two, three, four, 5, 6, 7, 8, 9, 10 or more nucleotide dimers, e.g. AT, AC, AG, TC, TG etc.; or nucleotide trimers or stretches of two, three, four, 5, 6, 7, 8, 9, 10 or more nucleotide trimers, e.g., ATC, TCG, ACG, ACC, AGG, TCC, TGG etc.

Further, alternative examples of parameters to be used for the construction of a virtual sequence are biophysical nucleic acid parameters. Such "biophysical nucleic acid parameters" include factors contributing to the nucleic acid conformation or structure, preferably measurable chemically or physically measurable values associated with nucleic acid sequence motives. Examples of such parameters are stacking energy, the presence of propeller twists, the bendability of the nucleic acid, the nucleic acid duplex stability, a disrupt energy associated with a specific nucleic acid fragment or motif, a free energy associated with a specific nucleic acid fragment or motif, the degree of duplex nucleic acid denaturation, e.g. DNA denaturation, and/or nucleic acid bending stiffness, e.g. DNA bending stiffness.

Corresponding parameters or values may be present in a virtual reference sequence in several suitable formats, e.g. linked to a conventional genomic sequence reference, linked to the subject they were obtained from, linked to specific chromosome identifiers etc.

The alignment to a reference sequence according to step (b) of the method according to the present invention as defined herein may, in preferred embodiments, be carried out with or based on a suitable reference alignment algorithm. Preferred examples of such algorithms include the algorithms BFAST, ELAND, GenomeMapper, GMAP, MAQ, MOSAIK, PASS, SeqMap, SHRiMP, SOAP, SSAHA, or CLD. Particularly preferred is the use of the algorithms Bowtie or BWA. Further envisaged is the combination of one or more of these algorithms. For example, a reference alignment may first be carried out with one of the mentioned algorithms, followed by a repetition by a different of these algorithms. Results of both procedures may be compared and, where appropriate, combined. It is, in general, preferred to use contiguous nucleotide sequences showing a minimum number of non-matching reads or non-aligned reads.

Details and ways of implementing these algorithms would be known to the person skilled in the art, or can be derived from suitable literature sources, e.g. from Bao et al., Journal of Human Genetics, 28 April 2011, p. 19. The present invention further envisages the use of optimized or further developed versions of these algorithms, or of reference alignment algorithms following a different scheme or algorithmic logic including not yet available algorithms, as long as the principle purpose of an alignment to a reference sequence as described herein is fulfilled.

In specific embodiments of the present invention step (b) of a method according to the present invention may be repeated once, twice or several times. The repetition of the sequence alignment may be carried out with the initially used fragments reads based on a different reference sequence, e.g. a closely related reference sequence derived from a different subject of the same species, or genus. Alternatively, a repetition of step (b) may be carried out on the basis of the results of a first run of method step (b), which are used a further template or reference sequence in addition to second reference sequence for a second run of method step (b).

In a third step of the method a gap or region of non-matching with the reference sequence may be detected. The terms "gap" and "region of non-matching" as used herein refer to a point or region within the newly assembled sequence, which shows no overlap with a reference sequence as defined herein, preferably with a genomic or sub-genomic reference sequence. Such a point or region showing no overlap may be of any suitable size, e.g. of a size of about 10 nucleotides to several thousand, ten thousand or more nucleotides. The size may vary in dependence on the reference sequence used. For the detection of a gap, a repetition of the reference sequence of method step (b) as defined herein may be carried out, e.g. based on one or more different reference sequence.

Upon the presence of a gap or point or region of non-matching after having performed method step (b) once, or, in specific embodiments, twice or more times, the aligning of nucleic acid data to a reference sequence may be stopped or halted and a *de novo* sequence assembly may be carried out in a further method step (d). In a specific embodiment of the present invention an alignment of the sequence data to a reference sequence may independently of the *de novo* assembly step be carried out for further sections of the genome, which show an overlap with regions of the reference sequence. A result of such a fragmented reference alignment procedure is the presence of aligned sequences interrupted by gaps or regions of non-matching. In certain embodiments said gaps may alternatively be filled with the help *of de novo* assembly algorithms as described herein.

The *de novo* assembly may accordingly be carried out with sequence data or nucleic acid fragment reads which are mapping to said gaps or regions of non-matching. The term "mapping to said gaps or regions of non-matching" means that at least one of the reads, i.e. an initial read shows a partial overlap with a sequence or sequence segment obtained with a reference sequence alignment, and also shows a sequence stretch which is not represented in the reference sequence. Further reads may partially overlap with said initial read and be arranged in a contiguous sequence spanning the entire gap or region of non-matching with the help of *de novo* assembly algorithms. In specific embodiments, in each or every 2^{nd}, 3^{rd}, 4^{th}, 5^{th}, 10^{th}, 15^{th}, 20^{th}, 30^{th} etc. round of read assembly a comparison or matching with a reference sequence may be performed in order to detect the end of a gap. In further embodiments, the gap may be closed from both directions, i.e. from the 5' and the 3' direction. In this embodiment, a checking for overlaps with the counter-directional sequence may be implemented.

In certain embodiments of the present invention, gaps may be closed sequentially, e.g. the reference sequence alignment algorithms may be performed until a gap is encountered. Subsequently, a *de novo* assembly step is performed, followed by a comparison with a reference sequence of the newly assembled stretch. In case an overlap with the reference sequence is encountered, the method is continued with reference sequence alignment step (b) until a further gap is encountered etc. This sequence of steps is performed until all or essentially all sequence data have been arranged, e.g. until 95%, 96%, 97%, 98%, 99%, 99.5%, 99.6%, 99.7%, 99.8%, 99.9%, 99.99% or 100% or the nucleic acid fragments reads have been arranged to contiguous nucleotide sequence segment(s).

In yet another preferred embodiment of the present invention, gaps may be closed by *de novo* assembly procedures simultaneously, e.g. after a simultaneous alignment of all sequence data to a reference sequence has been finished. Such a simultaneous closing may be carried out at all gaps, or at a sub-portion of encountered gaps.

The *de novo* assembly according to step (d) of the method according to the present invention as defined herein may, in preferred embodiments, be carried out with or based on a suitable *de novo* assembly algorithm. Preferred examples of such algorithms include the algorithms AAPATHS, Edena, EULER-SR, MIRA2, SEQAN, SHARCGS, SSAKE, SOAPdenovo, VCAKE. Particularly preferred is the use of the algorithms ABySS or Velvet. Further envisaged is the combination of one or more of these algorithms. For example, the *de novo* assembly may first be carried out with one of the mentioned algorithms, followed by a repetition by a different of these algorithms. In case sequence data could not be arranged, or the gaps could not be filled, the performance of the *de novo* assembly may be performed with one or more different algorithms.

Details and ways of implementing these algorithms would be known to the person skilled in the art, or can be derived from suitable literature sources, e.g. from Simpson et al., ABySS: a parallel assembler for short read sequence data, Genome Research, 2009, 19: 1117-1123 or from Zerbino et al., Velvet: Algorithms for de novo short read assembly using de Bruijn graphs, Genome Research, 2008, 18: 821-829. The present invention further envisages the use of optimized or further developed versions of these algorithms, or of *de novo* assembly algorithms following a different scheme or algorithmic logic including not yet available algorithms, as long as the principle purpose of a *de novo* assembly without guiding towards a reference sequence as described herein is fulfilled.

In an ultimate step (e) of the present method, the alignment output of the reference alignment of step (b) and of the *de novo* assembly of step (e) is combined. This combination typically results in a contiguous nucleotide sequence segment, or in contiguous nucleotide segments representing, for example, individual chromosomes, or individual sections of the genome, e.g. according to the regions of origin of the sequence data. Such a combination may be carried out continuously, e.g. after a gap has been filled by *de novo* assembly, a combined sequence segment may be produced, followed by a subsequent filling of a further gap etc. Alternatively, the combination may be performed simultaneously, e.g. when one or more gaps are filled at the same time. In such an embodiment, a further alignment step may be added, leading to an arrangement of the sequence of the gaps with the reference sequence obtained in step (b) of a method according to the present invention.

The resultant contiguous nucleotide sequence segment(s) may be stored or archived in any suitable format. A preferred storage format is the fasta or gbk format (which has genome annotation). Alternatively, the segment(s) may be stored in the SAM or BAM format, which has additional quality information which is important for further processing such as variant calling.

In yet another specific embodiment of the present in invention, the information on the contiguous nucleotide sequence segments(s) may be stored in a rapidly retrievable form. The storage of the genomic information may, for example, be limited to the available space on a suitable storage medium, e.g. a computer hard drive, a mobile storage device or the like. Particularly preferred is a storage structure which is 1) hierarchical, and/or 2) encodes specific sequencing information and/or additionally 3) contains links to patient data, images, reports etc. The term "rapidly retrievable" as used herein means that the sequence information is provided in a form, which allows an easy access to the information and/or allows an uncomplicated extraction of the stored information. Storage forms envisaged by the present invention are a suitable database storage, a storage in lists, numbered documents and/or in graphical form, e.g. as pictograms, graphical alignments, comparison schemes etc. In a specific embodiment of the present invention, the information may be combined with further information on the expression of genes, transcriptome analysis results, proteome analysis results, SNP analysis results, etc, and/or be retrieved from a storage medium and subsequently be displayed, e.g. on any suitable monitor, handheld device, computer device or the like.

In a further specific embodiment of the present invention, the detection or definition of one or more gaps or regions of non-matching regions may be carried out by implementing a filter or threshold. The term "implementing a filter or threshold" as used herein refers to a step of comparing sequence data or nucleic acid fragment reads with a predefined value or set of values in order to decide whether the sequence data represent a gap or region of non-matching. A corresponding filter or threshold may have or represent any suitable value allowing a distinction between sequence data or sequence reads capable of being aligned via a reference sequence alignment as defined herein, e.g. with the help of reference sequence alignment algorithms as defined herein, and, on the other hand, sequence data or sequence reads which are more efficiently assembled by *de novo* assembly as defined herein, e.g. with the help of *de novo* assembly algorithms as defined herein. In a specific embodiment, the filter or threshold values may be set in order to allow a prediction of a gap or a region of non-matching. Thereby, a proactive de novo assembly can be carried out.

In specific embodiments of the present invention, a filter or threshold may accordingly be implemented to distinguish between sequence data showing an acceptable base quality and a non-acceptable base quality. The term "acceptable base quality" as used herein refers to a phred-like quality score of about 20 and higher. A phred-like quality score is a Q score which is -10log₁₀(e) where e is the estimated probability of the base call being wrong. The method is typically used to measure the accuracy of sequencing data. Higher quality scores indicate a smaller probability that a base is called incorrectly. A quality score of 20 thus represents an error rate of 1 in 100, with a corresponding call accuracy of 99%. In further specific embodiments of the present invention a filter or threshold may be implemented to distinguish between sequence data showing an acceptable coverage and a non-acceptable coverage. The term "acceptable coverage" as used herein refers to a coverage of about 20X and above. Accordingly, the number of reads covering a base in an alignment is about 20, or more.

In further specific embodiments of the present invention, a filter or threshold may be implemented to distinguish between sequence data showing an acceptable high complexity of the surrounding region and a medium to low complexity of the surrounding region. The term "high complexity of the surrounding region" as used herein refers to the presence of repeated sequences stretches, e.g. the presence of repeated dimers, trimers, the presence of transposon remnants or repeated sequences derived from transposons etc.

In yet another specific embodiment of the present invention a filter or threshold may be implemented to distinguish between sequence data showing an acceptable length of mismatch and non-acceptable length of mismatch. The term "acceptable length of mismatch" as used herein refers to gaps that does not allow complete matching of a read to the reference sequence. A corresponding matching may be a continuous and non-continuous matching of about 70% and more. Corresponding filters or threshold may thus be used for the definition of a gap or break of reference sequence alignments as defined herein above. Upon the definition of a gap the performance of a *de novo* sequence assembly according to step (d) of the method of the present invention may be carried out.

In a further particularly preferred embodiment of the present invention the complexity and/or amount of the sequence data or nucleic acid fragments reads to be used as input for the reference sequence alignment of step (b) of a method according to the present invention may be reduced. The term "complexity" as used herein refers to the amount of variability of information present in the genomic sequence, the redundancy of sequence information present in the genomic sequence, the coverage of known chromosomal regions, genes, or spots of increased likelihood of mutation, as well as further parameters of genetic variability known to the person skilled in the art.

The "amount of genomic sequence" as used herein refers to parameters which restrict the available sequence data to specific portions or sub-portions of available data, e.g. which restrict the available sequence data to specific genome parts, specific chromosomes, specific chromosomal regions, genes, genetic elements, introns, exons, disease-associated regions or genes, biochemical pathways, genetic interaction pattern, expression pattern in dependence on the presence of growth factors, nutrients, hormones, cellular stress, transcription factor induction etc.

In certain embodiments of the present invention by reducing the complexity and/or amount of the sequence data thus certain aspects or sections of sequence data are masked out or excluded from entering into the present method, or alternatively, the sequence data or sequence information available is filtered for or focused on certain aspects. These aspects may preferably the localization in specific genome parts, on specific chromosomes, specific chromosomal regions, the presence of specific genes, genetic elements, introns, exons, disease-associated regions or genes, the association with specific biochemical pathways, the association with specific genetic interaction pattern, the association with specific expression pattern in dependence on the presence of growth factors, nutrients, hormones, cellular stress, transcription factor induction etc. Thus, for example, sequence data to be used for the method according to the present invention may specifically be filtered for only one specific gene, one chromosome, one chromosome region, etc. Further filter or focus parameters may be linked with known data on a disease, a group of diseases, a predisposition for a disease, e.g. a filter parameter may comprise all information on genomic modifications associated with a specific disease, group of diseases or predisposition for the disease. Accordingly, in a further specific embodiment of the present invention the amount of sequence data may be reduced to genomic regions, whole genes, exons (the exome sequence), transcription factor binding sites, DNA methylation-binding-protein binding sites, intergenic regions which may include short or long non-coding RNAs, etc. which are known or suspected to be clinically relevant or important and might be variable or highly variable between human beings, between different human races, or populations, between the human or animal sexes, between age groups of human beings, e.g. between newborn babies and adults, between human beings and other organisms etc., between animals of the same race, between animals of different races, species, genera or classes, between plant varieties, plant species etc., or which are known or suspected to be variable or highly variable in diseases or disorders. Such genomic regions, genes, exons, binding sites etc. would be known to the person skilled in the art or could be derived from suitable textbooks or information repositories, e.g. from the UCSC genome browser or from NCBI. A reduction of the complexity and/or amount of the sequence data may be based on methods described in suitable textbooks or scientific documents such as S. Kurtz, A. Phillippy, A.L. Delcher, M. Smoot, M. Shumway, C. Antonescu, and S.L. Salzberg, 2004, "Versatile and open software for comparing large genomes", Genome Biology, 5:R12, Schuster et al., 2010, Nature 463(18), 943-947 or Fujimoto et al, 2010, Nature Genetics, 42, 931-936. Further envisaged methods for the reduction of the complexity and/or amount of the genomic sequence may be derived from Ashley et al., 2010, The Lancet, 375, 1525-1535.

In a further specific embodiment, a reduction of the complexity and/or amount of the sequence data may be based on the information provided by the Pharmacogenomic Knowledge Base (PharmGKB) with respect regard to drug-response phenotypes, the locus-specific mutation database (LSMD) or the human mitochondrial genome polymorphism database (mtSNP). Also envisaged is the employment of population-based filters for the sequence data. Thus, if for instance there is a variant SNP known for a specific population, race, age group etc., this variant may not be reported or identified as relevant or filtered out for the purpose of the present invention. In specific embodiments, such variants may - although being specific or typical for a population, race, age group etc - be considered and identified as relevant for the purpose of the present invention, if the variant shows an important/clinical functional implication. An example of a functionally important class of SNPs, which may appear in a whole population is in the CYP related genes which help to metabolize and excrete drugs. Since certain drugs are known to be tolerated at a different, e.g. lower dosages in different populations, e.g. in non-Caucasian), variants in CYP-related genes may be filtered, sorted, classified and/or assessed in accordance with the patient's population affiliation, or the patient's race. Such a filtering may, for example, be carried out on the basis of information provided in the PharmGKB database.

These aspects may, in further preferred embodiments, be defined individually before a method is carried out, e.g. based on information derived from the literature concerning known genomic signatures or polymorphic sites in association with diseases and its mapping onto a reference sequence.

In further embodiments, the method steps, threshold values and/or filter values may also be individualized or adapted to specific situations, e.g. to specific diseases, specific genomes, specific organisms, or any other feature. This may, for example, be carried out by providing the user with a possibility of defining a mask, a threshold, a reference sequence etc.

The above outlined embodiments thus enable the skilled person to streamline the assembly and alignment process to sectors of the genome or to specific datasets associated with specific molecular or medicinal questions or problems. Accordingly filtered sequence datasets may be entered into a method according to the present invention, in particular to a reference sequence alignment step as defined in step (b). Subsequently, upon the encountering of gaps or regions of non-matching as defined herein above or below a *de novo* assembly according to step (d) may be carried out.

In further embodiments of the present invention, by reducing the complexity and/or amount of the sequence data certain aspects or sections of sequence data are masked out or excluded from entering into the reference sequence alignment of step (b) of the present method according to different suitable parameters, but may subsequently be used for a different step of the method, or may be used for both, step (b) or step (e) of a method of the present invention. These aspects are preferably the presence of intergenic regions, the presence of polymorphisms, the presence of introns or exons, the presence of transposable elements, the presence of repetitive elements, the presence of spots or regions of known mutations. Particularly preferred in this context is a masking out of nucleic acid sequence data relating to known polymorphisms, highly variable regions, disease related mutations or modifications, sequence repeats, low mapability regions, CPG islands, or regions with specific biophysical features.

The term "known polymorphisms" as used herein refers to the presence of single nucleotide polymorphisms, or dimer or trimer polymorphisms in sequence data. For example, the presence of sequence data known to be associated to polymorphic sites, e.g. known to comprise polymorphic repeats, or different single nucleotide polymorphism, may lead to a masking out of the sequence data.

The term "highly variable regions" as used herein relates to the presence of sequence stretches which are known to be highly variable, e.g. transposon elements, Alu-sequences, elements conventionally used for DNA-fingerprinting etc. For example, the presence of sequence data known to be associated to highly variable regions may lead to a masking out of the sequence data.

The term "disease related mutations or modifications" relates to any known modification of the genome, which can contribute to a disease. Preferred are diseases based on sequence modifications comprising larger DNA rearrangements, deletions, insertions or other mutations. In certain embodiments, the presence of sequence data known to be associated to the presence of disease related mutations or modifications may lead to a masking out of the sequence data. In further embodiments, correspondingly identified sequence data pieces may be tagged and included into the reference sequence alignment and additionally be used for *de novo* assembly. The *de novo* assembled sequence may subsequently be compared to the reference alignment. In case of a difference in both alignment procedures, an additional multiple sequence alignment with a different algorithm may be carried out and the results of both the alignments may be combined keeping flanking sequence in consideration.

"Sequence repeats" as used herein refers to any repetition of sequences known to the person skilled in the art, e.g. the presence of inverted repeats, non-inverted repeats, dimer or trimer repeats, the presence of copies of a gene or genomic region, the presence of pseudogenes, the rearrangement of entire chromosomal regions or arms etc.

The term "low mapability region" as used herein refers to sequences which are present in a lower coverage than the average sequence reads. A low mapability region may, for example, be present if the coverage of the reads is reduced in comparison to the average coverage by a factor of 50%, 100%, 150%, 200%, 300%, 400%, 500% or more. In certain embodiments, the presence of sequence data known to be associated to the presence of low mapability regions may lead to a masking out of the sequence data. In further embodiments, correspondingly identified sequence data pieces may be tagged and included into the reference sequence alignment and additionally be used for *de novo* assembly. The *de novo* assembled sequence may subsequently be compared to the reference alignment. In case of a difference in both alignment procedures, an additional multiple sequence alignment with a different algorithm may be carried out, and the results of both the alignments will be combined keeping flanking sequence in consideration.

The term "CPG islands" as used herein refers to sequences containing a high frequency of CpG sites, which are typically 300-3,000 base pairs in length and are frequently in the proximity of promoters. In certain embodiments, the presence of sequence data known to be associated to the presence of CPG islands may lead to a masking out of the sequence data. In further embodiments, correspondingly identified sequence data pieces may be tagged and included into the reference sequence alignment and additionally be used for *de novo* assembly. The *de novo* assembled sequence may subsequently be compared to the reference alignment. In case of a difference in both alignment procedures, an additional multiple sequence alignment with a different algorithm may be carried out, and the results of both the alignments will be combined keeping flanking sequence in consideration.

The term "specific biophysical features" as used herein refers to the presence of propeller twists, a high or low degree of bendability of the nucleic acid, a high or low degree of duplex stability, a high or low amount of disrupt energy associated with nucleic acid fragments or reads, a high or low amount of free energy associated with nucleic acid fragments or reads, a high or low degree of DNA denaturation or a high or low degree of DNA bending stiffness. The terms "high or low degree" or "high or low amount" as used herein refer to states of the nucleic acids, which differ from the average or standard values typical for nucleic acids fragments, in particular if calculated for single nucleic acids reads as defined herein. Such a difference may be a difference of about 5%, 10%, 15%, 20%, 25%, 30%, 35% or more. In certain embodiments, the presence of sequence data known to be associated to the presence of specific biophysical features as defined herein may lead to a masking out of the sequence data. In further embodiments, correspondingly identified sequence data pieces may be tagged and included into the reference sequence alignment and additionally be used for *de novo* assembly. The *de novo* assembled sequence may subsequently be compared to the reference alignment. In case of a difference in both alignment procedures, an additional multiple sequence alignment with a different algorithm may be carried out, and the results of both the alignments will be combined keeping flanking sequence in consideration.

The term "multiple sequence alignment" as used herein refers to the alignment of three or more biological sequences, e.g. DNA, RNA, protein etc. For the performance of a multiple sequence alignment any suitable algorithm may be used, e.g. algorithms capable of comparing and identifying the similarity among multiple sequences. The algorithms may preferably use gapped alignment heuristics. A preferred algorithm for multiple sequence alignment is ClustalW.

The above defined sequence features may, for example, be implemented by a correspondingly defined score, or a threshold decision, or, in other embodiments, by a specific detection of known pattern or sequence motifs. In all these cases, the detection of a motif or feature as defined herein may contribute to a masking out of the sequence data. In addition, the masked out sequence data may be tagged for a different assembly or alignment process.

In a further preferred embodiment of the present invention, a masking out of sequence data may be based on the conservation of sequences, e.g. be implemented by a conservation score. For example, highly conserved regions of the genome may be entered into a reference sequence alignment procedure, whereas a low conservation may tag the sequence data for a *de novo* assembly.

In a further preferred embodiment of the present invention said masked out nucleic acid sequence data as defined herein above may be subjected to a *de novo* sequence assembly according to step (d) of the present method. For example, nucleic acid sequence data relating to known polymorphisms, highly variable regions, disease related mutations or modifications, sequence repeats, low mapability regions, CPG islands, or regions with specific biophysical features as defined herein above may be used directly for a *de novo* sequence assembly. In further embodiments, these data may be employed for both, a *de novo* assembly and a reference sequence alignment.

In a specific embodiment of the present invention the following method steps are carried out: An algorithm may be provided in a shortlisted form and configured to run in parallel, comprising the following steps which will be performed on data in a standard, preferably unified format, as defined herein above:
(1) A reference sequence alignment is started.
(2) A gap is detected or the place where reference assembly breaks is identified or i bases in multiple reads that doesn't match with the reference are identified. In order to perform this step a threshold of base quality and coverage is defined. Reads passing this threshold will be considered for defining a gap or break. Additionally or alternatively, a length of mismatch of the read with reference may be set as defining point for the gap or break.
(3) A landmark of known reported polymorphisms (SNP/ indel/ CNV, etc) is provided before hand to the aligner in order to check stringently at those specific regions in the genome.
(4) Once the gap/ break/ mismatch is defined the *de novo* alignment will come in action.
(5) *De novo* alignment is carried out for the region of the gap. Again here a threshold of base quality and coverage is set to determine a good assembly. This will be continued until the next point where read bases start matching with the reference bases.
(6) Once this step is identified, reference alignment is performed again from that region on forward.
(7) These steps are repeated until the complete assembly is finished.
(8) Subsequently, the alignment results are stored in a format that is easy for SNP/ indel/ other variant calling from the alignment results, e.g in a SAM or BAM format.

In a further preferred embodiment, all method steps as depicted in Fig. 4 may be carried out. In further embodiments, also a subset of these steps may be carried out in dependence of the quality of the sequence data, the necessity for performing certain steps etc.

In a further aspect the present invention relates to a program element or computer program or a software for assembly of nucleic acid sequence data comprising nucleic acid fragment reads into contiguous nucleotide sequence segments, which when being executed by a processor is adapted to carry out the steps of the method as defined herein above. The program element or computer program or a software may, in one embodiment, implement a reference alignment algorithm and a *de novo* assembly algorithm as defined herein. In further embodiments, threshold and filter parameters as defined herein above may be additionally implemented. In further embodiments, specific comparison steps, storage activities, format conversion steps etc. may be additionally or alternatively be implemented. The implementation may be provided for any suitable computer platform or be based on any suitable programming language known to the person skilled in the art.

In a further aspect the present invention relates to a sequence assembly system for transforming nucleic acid sequence data comprising nucleic acid fragment reads into (a) contiguous nucleotide sequence segment(s), comprising a computer processor, memory, and (a) data storage device(s), the memory having programming instructions to execute a program element or computer program as defined herein above. In specific embodiments of the present invention, said sequence assembly system may be equipped with additional input and output interfaces, e.g. to be used in a network, or to be used as standalone version. In a further embodiment, it may also comprise an input or user interface allowing the definition or adaption of threshold or filter values, the indication or modification of reference sequences, the focusing on genome portions or specific aspects etc. In further embodiments, it may be connected to a database or a database server, or be connected to an in-house, intranet or internet server for providing client computers or users with assembly results. The data storage device as mentioned herein may be any suitable data storage device such as a hard drive, an optical drive, a server as defined herein above etc.

In further embodiments of the present invention, outputted resulting data may accordingly be stored in any suitable manner or format, preferably in a storage structure, which is 1) hierarchical, and/or 2) encodes specific sequencing information and/or additionally 3) contains links to patient data, images, reports etc.

In a further preferred embodiment of the present invention the sequence assembly system as defined herein above may be associated or connected to a sequencer device. For example, the sequence assembly system as defined herein may be associated or connected to a Roche/454, an Illumina/Solex and an ABI SOLiD system, or to further advanced developments of these sequencer devices, or to any other suitable sequencer device, including devices not yet available to the skilled person.

In yet another preferred embodiment of the present invention the sequence assembly system as defined herein above may be associated or connected to a diagnostic decision support system. A "diagnostic decision support system" as used herein refers to system comprising an input for providing a subject's genomic or sub-genomic sequence data and, in specific embodiments, optionally its functional readout, for example gene or non-coding RNA expression, or protein levels. In addition, the system comprises a program element or computer program or a software for assembly of nucleic acid sequence data comprising nucleic acid fragment reads into contiguous nucleotide sequence segments, which when being executed by a processor is adapted to carry out the steps of the method as defined herein above, and an output for outputting a subject's contiguous nucleotide sequence segment(s) variation, and a medium for storing the outputted information. Preferably, the outputted information is able to indicate the presence or absence of genomic modifications, more preferably the affliction of a subject by a disease or a predisposition for a disease.

The disease or disorder which may be detected or diagnosed or prognosticated according to the present invention may be any detectable disease known to the person skilled in the art. In a preferred embodiment said disease may be a genetic disease or disorder, in particular a disorder, which can be detected on the basis of genomic sequence data. Such disorders include, but are not limited to, the disorders mentioned, for example, in suitable scientific literature, clinical or medical publications, qualified textbooks, public information repositories, internet resources or databases, in particular one or more of those mentioned in http://en.wikipedia.org/wiki/List_of_genetic_disorders.

In a particularly preferred embodiment of the present invention said disease is a cancerous disease, e.g. any cancerous disease or tumor known to the person skilled in the art. More preferably, the disease is breast cancer, ovarian cancer, or prostate cancer.

In a specific embodiment said diagnostic decision support system may be a molecular oncology decision making workstation. The decision making workstation may preferably be used for deciding on the initiation and/or continuation of a cancer therapy for a subject. Further envisaged are similar decision making workstation for different disease types, e.g. for any of the diseases as mentioned herein above.

In yet another embodiment of the present invention, the diagnostic decision support system may be an electronic picture/data archiving and communication system.

The following example and figures are provided for illustrative purposes. It is thus understood that the example and figures are not to be construed as limiting. The skilled person in the art will clearly be able to envisage further modifications of the principles laid out herein.

### EXAMPLES

### Example 1 - Reference and de novo alignment of the sequence reads to establish the exact repeat content of the AVPR1A gene

Since the repeat content (number of repeat) of AVPR1A gene is related to behavior, it has significant health implication. Accordingly, an experimental evaluation was carried on the basis of a reference and *de novo* alignment of the sequence reads to establish the exact repeat content of the AVPR1A gene.

Reference alignment was used for mapping the reads to the genomic coordinates and *de novo* for determining the exact repeat content in AVPR1A gene (see Figs. 5 and 6).

Qseq files obtained from Illumina GAIIx were first converted into fastq format. These files were then aligned to a human reference (GRCh37) genome using BWA aligner. A consensus sequence was built using SAM output from BWA alignment. We know that RS3 polymorphism in AVPR1 gene is highly polymorphic in nature and is associated with clinical phenotype, so we extracted the reads from same chromosome and performed *de novo* alignment using ABySS. This generated contigs of various sizes. Subsequently multiple sequence alignment was performed using ClustalW of the contigs and the AVPR1 gene. This alignment allowed gaps and mismatches. It was observed that the contigs obtained using *de novo* alignment had different RS3 repeat. This additional repeat content was subsequently inserted in the reference consensus by keeping flanking sequence into consideration. This lead to the generation of a consensus sequence that has different repeat length when compared to reference.

A reference sequence alignment approach alone could not reveal the exact number of repeat content (i.e. 24 repeats), while the application of a de novo assembly algorithm could established it. A combination of both algorithms was capable of correctly mapping the sequences to reference genomic coordinates with an exact repeat content.

## Claims

1. A computer-implemented method for assembly of nucleic acid sequence data comprising nucleic acid fragment reads into (a) contiguous nucleotide sequence segment(s), comprising the steps of:
(a) obtaining a plurality of nucleic acid sequence data from a plurality of nucleic acid fragment reads;
(b) aligning said plurality of nucleic acid sequence data to a reference sequence;
(c) detecting one or more gaps or regions of non-matching with the reference sequence in the alignment output of step (b), wherein prior to the aligning step (b) a masking out of nucleic acid sequence data relating to known polymorphisms, highly variable regions, disease related mutations or modifications, repeats, low mapability regions, CPG islands, or regions with specific biophysical features is performed;
(d) performing *de novo* sequence assembly of nucleic acid sequence data mapping to said gaps or regions of non-matching; and
(e) combining the alignment output of step (b) and the assembly output of step (d) in order to obtain (a) contiguous nucleotide sequence segment(s).

2. The method of claim 1, wherein said plurality of nucleic acid sequence data is converted to a unified format.

3. The method of claim 1 or 2, wherein said detection of step (c) is performed by implementing a filter or threshold.

4. The method of claim 3, wherein said filter or threshold is a base quality, coverage, complexity of the surrounding region or length of mismatch filter or threshold.

5. The method of claim 1, wherein said masked out nucleic acid sequence data is subjected to a *de novo* sequence assembly of step (d).

6. The method of any one of claims 1 to 5, wherein step (b) is carried out with a reference alignment algorithm, preferably with BFAST, ELAND, GenomeMapper, GMAP, MAQ, MOSAIK, PASS, SeqMap, SHRiMP, SOAP, SSAHA, or CLD, more preferably with Bowtie or BWA.

7. The method of any one of claims 1 to 6, wherein step (c) is carried out with a *de novo* assembly algorithm, preferably with AAPATHS, Edena, EULER-SR, MIRA2, SEQAN, SHARCGS, SSAKE, SOAPdenovo, VCAKE, more preferably with ABySS or Velvet.

8. The method of any one of claims 1 to 7, wherein said reference sequence is an essentially complete prokaryotic, eukaryotic or viral genome sequence, or a sub-portion thereof, preferably a human genome sequence, an animal genome sequence, a plant genome sequence, a bacterial genome sequence, or a sub-portion thereof.

9. The method of claim 8, wherein said reference sequence is selected from a group or taxon, which is phylogenetically related to the organism, whose nucleic acid sequence data is to be assembled.

10. The method of claim 8, wherein said reference sequence is a genomic sub-portion having regulatory potential selected from the group comprising exon sequences, promoter sequences, enhancer sequences, transcription factor binding sites, or any grouping or sub-grouping thereof.

11. The method of any one of claims 1 to 10, wherein said reference sequence is a virtual sequence based on sequence composition parameters, such as the presence of monomers, dimers and/or trimers, or based on biophysical nucleic acid properties, such as stacking energy, propeller twist, bendability, duplex stability, disrupt energy, free energy, DNA denaturation or DNA bending stiffness.

12. A computer program for assembly of nucleic acid sequence data comprising nucleic acid fragment reads into contiguous nucleotide sequence segments, which when being executed by a processor is adapted to carry out the steps of the method of any one of claims 1 to 11.

13. A sequence assembly system for transforming nucleic acid sequence data comprising nucleic acid fragment reads into (a) contiguous nucleotide sequence segment(s), comprising a computer processor, memory, and (a) data storage device(s), the memory having programming instructions to execute the computer program according to claim 13.

14. The system of claim 13, which is associated or connected to a sequencer device, or which is a medical decision support system, preferably a diagnostic decision support system.

## Patentansprüche

1. Computerimplementiertes Verfahren zur Assemblierung von Nukleinsäure-Sequenzdaten, umfassend Nukleinsäure-Fragmentauslesungen in (a) eines oder mehrere zusammenhängende Nucleotidsequenzsegmente, umfassend die Schritte:
(a) Erhalten einer Vielzahl von Nukleinsäure-Sequenzdaten aus einer Vielzahl von Nukleinsäure-Fragmentauslesungen;
(b) Ausrichten der Vielzahl von Nukleinsäure-Sequenzdaten mit einer Referenzsequenz;
(c) Detektieren einer oder mehrerer Lücken oder Regionen in Nichtübereinstimmung mit der Referenzsequenz in der Ausrichtungsausgabe von Schritt (b), wobei vor dem Ausrichtungsschritt (b) eine Ausblendung von Nukleinsäure-Sequenzdaten, die sich auf bekannte Polymorphismen, hochvariable Regionen, krankheitsbezogene Mutationen oder Modifikationen, Repeats, Regionen mit geringer Abbildbarkeit, CPG-Inseln oder Regionen mit spezifischen biophysikalischen Merkmalen beziehen, durchgeführt wird;
(d) Durchführen einer De-novo-Sequenz-Assemblierung der Nukleinsäure-Sequenz-Datenabbildung an den Lücken oder Regionen der Nichtübereinstimmung; und
(e) Kombinieren der Ausrichtungsausgabe von Schritt (b) und der Assemblierungsausgabe von Schritt (d), um (a) eines oder mehrere zusammenhängende Nucleotidsequenzsegmente zu erhalten.

2. Verfahren nach Anspruch 1, wobei die Vielzahl von Nukleinsäure-Sequenzdaten in ein einheitliches Format umgewandelt wird.

3. Verfahren nach Anspruch 1 oder 2, wobei die Detektion von Schritt (c) durch Implementieren eines Filters oder Schwellenwerts durchgeführt wird.

4. Verfahren nach Anspruch 3, wobei der Filter oder der Schwellenwert eine Basisqualität, eine Abdeckung, eine Komplexität der umgebenden Region oder eine Länge des Nichtübereinstimmungsfilters oder Schwellenwerts ist.

5. Verfahren nach Anspruch 1, wobei die ausgeblendeten Nukleinsäure-Sequenzdaten einer De-Novo-Sequenzassemblierung von Schritt (d) unterzogen werden.

6. Verfahren nach einem der Ansprüche 1 bis 5, wobei Schritt (b) mit einem Referenzausrichtungsalgorithmus ausgeführt wird, vorzugsweise mit BFAST, ELAND, GenomeMapper, GMAP, MAQ, MOSAIK, PASS, SeqMap, SHRiMP, SOAP, SSAHA oder CLD, mehr bevorzugt mit Bowtie oder BWA.

7. Verfahren nach einem der Ansprüche 1 bis 6, wobei Schritt (c) mit einem De-novo-Assemblierungsalgorithmus durchgeführt wird, vorzugsweise mit AAPATHS, Edena, EULER-SR, MIRA2, SEQAN, SHARCGS, SSAKE, SOAPdenovo, VCAKE, mehr bevorzugt mit ABySS oder Velvet.

8. Verfahren nach einem der Ansprüche 1 bis 7, wobei die Referenzsequenz eine im Wesentlichen vollständige prokaryotische, eukaryotische oder virale Genomsequenz oder ein Teilabschnitt davon ist, vorzugsweise eine menschliche Genomsequenz, eine tierische Genomsequenz, eine pflanzliche Genomsequenz, eine bakterielle Genomsequenz oder ein Teilabschnitt davon.

9. Verfahren nach Anspruch 8, wobei die Referenzsequenz ausgewählt ist aus einer Gruppe oder einem Taxon, das mit dem Organismus phylogenetisch verwandt ist, dessen Nukleinsäure-Sequenzdaten assembliert werden sollen.

10. Verfahren nach Anspruch 8, wobei die Referenzsequenz ein Genomteilabschnitt mit regulatorischem Potenzial ist, ausgewählt aus der Gruppe, umfassend Exonsequenzen, Promotorsequenzen, Enhancersequenzen, Transkriptionsfaktorbindungsstellen oder jede beliebige Gruppierung oder Untergruppierung davon.

11. Verfahren nach einem der Ansprüche 1 bis 10, wobei die Referenzsequenz eine virtuelle Sequenz ist, basierend auf Sequenzzusammensetzungsparametern, wie der Anwesenheit von Monomeren, Dimeren und/oder Trimeren oder basierend auf biophysikalischen Nukleinsäureeigenschaften, wie zum Beispiel Stapelenergie Propeller-Twist, Biegbarkeit, Duplex-Stabilität, Störenergie, freie Energie, DNA-Denaturierung oder DNA-Biegesteifigkeit.

12. Computerprogramm zum Assemblieren von Nukleinsäure-Sequenzdaten, umfassend Nukleinsäure-Fragmentauslesungen zu zusammenhängenden Nucleotidsequenzsegmenten, das, wenn es von einem Prozessor ausgeführt wird, zum Ausführen der Verfahrensschritte nach einem der Ansprüche 1 bis 11 ausgelegt ist.

13. Sequenzassembliersystem zum Umwandeln von Nukleinsäure-Sequenzdaten, umfassend Nukleinsäure-Fragmente, in (a) eines oder mehrere zusammenhängende Nucleotidsequenzsegmente, umfassend einen Computerprozessor, Speicher und (a) eine oder mehrere Datenspeichervorrichtungen, wobei der Speicher Programmierungsanweisungen aufweist zur Ausführung des Computerprogramms nach Anspruch 13 aufweist.

14. System nach Anspruch 13, das einer Sequenzervorrichtung zugeordnet oder damit verbunden ist oder das ein System zur Unterstützung medizinischer Entscheidungen ist, vorzugsweise ein System zur Unterstützung von Diagnoseentscheidungen.

## Revendications

1. Procédé mis en oeuvre par ordinateur pour l'assemblage de données de séquence d'acide nucléique comprenant des lectures de fragments d'acide nucléique dans a) un ou plusieurs segments de séquence nucléotidique contigus, comprenant les étapes suivantes :
a) obtention d'une pluralité de données de séquence d'acide nucléique provenant d'une pluralité de lectures de fragments d'acide nucléique ;
b) alignement de ladite pluralité de données de séquence d'acide nucléique sur une séquence de référence ;
c) détection d'un ou plusieurs espaces ou d'une ou plusieurs régions de non-correspondance avec la séquence de référence dans la sortie d'alignement de l'étape b), dans lequel avant l'étape d'alignement b) on procède au masquage des données de séquence d'acide nucléique concernant des polymorphismes connus, des régions extrêmement variables, des mutations ou des modifications liées à la maladie, des répétitions, des régions de faible aptitude à la cartographie, des îlots CPG, ou des régions à caractéristiques biophysiques spécifiques ;
d) réalisation d'un assemblage de séquence *de novo* de données de séquence d'acide nucléique se cartographiant auxdits espaces ou auxdites régions de non-correspondance ; et
e) combinaison de la sortie d'alignement de l'étape b) et de la sortie d'assemblage de l'étape d) afin d'obtenir a) un ou plusieurs segments de séquence nucléotidique contigus.

2. Procédé selon la revendication 1, dans lequel ladite pluralité de données de séquence d'acide nucléique est convertie en un format unifié.

3. Procédé selon les revendications 1 ou 2, dans lequel ladite détection de l'étape c) est réalisée par mise en oeuvre d'un filtre ou d'un seuil.

4. Procédé selon la revendication 3, dans lequel ledit filtre ou seuil correspond à la qualité de base, la couverture de base, la complexité de base de la région environnante ou à la longueur d'un filtre ou d'un seuil de disparité.

5. Procédé selon la revendication 1, dans lequel lesdites données de séquence d'acide nucléique masquées sont soumises à un assemblage de séquence *de novo* selon l'étape d).

6. Procédé selon l'une quelconque des revendications 1 à 5, dans lequel l'étape b) est effectuée avec un algorithme d'alignement de référence, de préférence avec BFAST, ELAND, GenomeMapper, GMAP, MAQ, MOSAIK, PASS, SeqMap, SHRiMP, SOAP, SSAHA ou CLD, de préférence avec Bowtie ou BWA.

7. Procédé selon l'une quelconque des revendications 1 à 6, dans lequel l'étape c) est effectuée avec un algorithme d'assemblage *de novo,* de préférence avec AAPATHS, Edena, EULER-SR, MIRA2, SEQAN, SHARCGS, SOAPdenovo, VCAKE, de préférence avec ABySS or Velvet.

8. Procédé selon l'une quelconque des revendications 1 à 7, dans lequel ladite séquence de référence est une séquence de génome procaryote, eucaryote ou viral essentiellement complète, ou une sous-partie de celle-ci, de préférence une séquence de génome humain, une séquence de génome animal, une séquence de génome végétal, une séquence de génome bactérien ou une sous-partie de celle-ci.

9. Procédé selon la revendication 8, dans lequel ladite séquence de référence est choisie dans un groupe ou un taxon qui est lié phylogénétiquement à l'organisme dont les données de séquence d'acide nucléique doivent être assemblées.

10. Procédé selon la revendication 8, dans lequel ladite séquence de référence est une sous-partie génomique dotée d'un potentiel de régulation et choisie dans le groupe comprenant les séquences exon, les séquences promoteur, les séquences amplificateur, les sites de liaison de facteurs de transcription, ou un quelconque regroupement ou sous-regroupement de celles-ci.

11. Procédé selon l'une quelconque des revendications 1 à 10, dans lequel ladite séquence de référence est une séquence virtuelle basée sur des paramètres de composition de séquence, tels que la présence de monomères, de dimères et/ou de trimères, ou basée sur des propriétés biophysiques d'acide nucléique, telles que l'énergie d'empilement, la torsion d'hélice, l'aptitude au repliement, la stabilité duplex, l'énergie de perturbation, l'énergie libre, la dénaturation de l'ADN ou la rigidité de repliement de l'ADN.

12. Programme informatique pour l'assemblage de données de séquence d'acide nucléique comprenant des lectures de fragments d'acide nucléique dans des segments de séquence nucléotidique contigus lequel, lorsqu'il est exécuté par un processeur, est adapté pour effectuer les étapes du procédé selon l'une quelconque des revendications 1 à 11.

13. Système d'assemblage de séquence pour transformer des données de séquence d'acide nucléique comprenant des lectures de fragments d'acide nucléique en a) un ou plusieurs segments de séquence nucléotidique contigus, comprenant un processeur d'ordinateur, une mémoire et a) un ou plusieurs périphériques de stockage de données, la mémoire contenant des instructions de programmation pour exécuter le programme informatique selon la revendication 13.

14. Système selon la revendication 13 qui est associé ou relié à un dispositif séquenceur ou qui est un système d'aide à la décision médicale, de préférence un système d'aide à la décision de diagnostic.
